# EUROPEAN PATENT APPLICATION

(11) **EP 3 530 661 A1**
(43) Date of publication of application: **28.08.2019**
(21) Application number: 18158380.8
(22) Date of filing: 23.02.2018
(51) Int. Cl.: C07D 475/04, G01N 33/82

(54) **METHOD AND KIT FOR ISOTOPE-LABELLING OF A FOLATE-CONTAINING BIOLOGICAL SAMPLE FOR MASS SPECTROMETRY**

(71) Applicant: Medizinische Universität Graz, 8036 Graz (AT)
(72) Inventor: SCHITTMAYER-SCHANTL, Matthias, 8036 Graz (AT); BIRNER-GRÜNBERGER, Ruth, 8036 Graz (AT)
(74) Representative: Sonn & Partner Patentanwälte

(57) **Abstract**

The present invention provides a method of stabilising a biological sample comprising at least two folate species. The method comprises providing the biological sample and contacting the folate species with a reducing agent and an aliphatic aldehyde or ketone under conditions which allow reductive alkylation of at least one of the folate species, wherein the reducing agent is a deuterated reducing agent, such as formaldehyde-¹³C, D₂, and the aliphatic aldehyde or ketone is a deuterated aliphatic aldehyde or ketone, such as cyanoborodeuteride, whereby at least one stabilised folate species isotopically labelled with D is obtained. Also provided is a biological sample containing at least one stabilised folate species selected from the group consisting of compounds I-VIII, as well as a mass-spectrometric method for quantifying folate species in the biological sample and a kit for stabilising a biological sample comprising at least two folate species for mass spectrometric analysis.

## Description

The field of present invention relates to methods of stabilising and isotope-labelling a biological sample comprising at least two folate species, in particular for subsequent mass spectrometric analysis, as well as kits for this purpose.

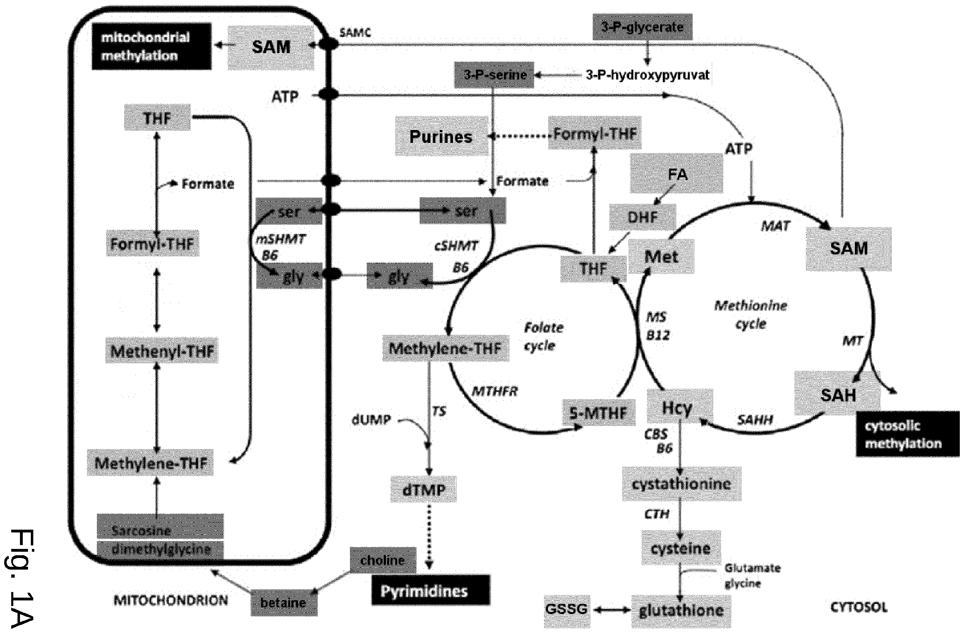

One-carbon (C1) metabolism is a central metabolic pathway which distributes C1 units derived from C1 donors to the crucial cellular pathways purine synthesis, thymidine synthesis and the S-adenosyl methionine cycle (see also Fig. 1A). Alterations in C1 metabolism have been reported in numerous diseases, including neural tube defects (Narisawa et al.), cardiovascular disease (Li et al.) and cancer (Assaraf; Locasale). In mammals, potential C1 donors include the non-essential amino acids serine and glycine, the essential amino acids histidine and tryptophan as well as the degradation products of choline, betaine, dimethylglycine and N-methylglycine (Locasale; Newman & Maddocks).

Folates are essential co-factors of C1 metabolism. They act as carriers which can temporarily bind C1 groups in different oxidation states and comprise pteridine, p-aminobenzoic acid and glutamic acid (forming a polyglutamate tail) as building blocks. Folate species include 5-methyl tetrahydrofolate (THF), 5,10-methylene THF, 5,10-methenyl THF, 5-formyl THF, 10-formyl THF, dihydrofolic acid (DHF) and folic acid (FA). Folate species are thought to exist in large part as stabilized, protein bound form in cells and some are unstable in solution. Quantifying intracellular folate pools, which is important for studying and diagnosing diseases such as the ones mentioned above, typically requires quenching of enzymatic activity and extraction of folates from their native protein environment which can result in loss of analytes.

Traditionally, the first step of folate analytics used to be enzymatic deconjugation, i.e. the trimming of the polyglutamate tail until only one glutamate is left (Jägerstad et al.; Freisleben et al.). While this approach requires less sensitive analytical methods since it pools several glutamation states into one, it also sacrifices valuable information, for example the "domino effect" reported by Kwon et al. is not observable using this approach. Another problem is the lengthy enzymatic deglutamation step, which promotes interconversion and potential loss of unstable folate species. Especially pteridine ring oxidation and subsequent bond cleavage between the pteridine ring and para-Aminobenzoyl glutamate has been reported as one major pathway of folate degradation (Reed & Archer). Finally, the source of folate conjugase which is in most cases a crude preparation (i.e. charcoal treated) of rat plasma is a potential source of sample contamination given the low hydrophobicity of even monoglutamated folates. With the availability of highly sensitive liquid chromatography (LC)-mass spectrometry (MS) methods, the compromises made by deconjugation have become unnecessary. However, even methods avoiding deconjugation suffer from the low chemical stability of several folate species, making their analysis a highly challenging task.

Several LC-MS methods were published attempting to quantify folate pools individually, most of them focusing on the more stable folate species or employing isotope dilution approaches to account for losses during sample preparation and analysis (Garratt et al.; Lu et al.; Haandel et al.; Ringling et al., 2017; Kiekens et al. 2015; Ringling et al. 2013; WO 2010/006070 A1). However, isotope dilution cannot compensate for interconversion reactions taking place between folate pools where less stable folate species are converted to folate species which are more stable under extraction or analytical conditions and add to those pools. In other words, the methods in the prior art do not provide the complete picture of physiological folate pools.

Chen et al. relates to an LC-MS chemical derivatization method for the measurement of five different one-carbon states of cellular THF. In this method, chemical reduction of methylene-THF using deuterated sodium cyanoborohydride traps methylene-THF, which is unstable, as deuterated 5-methyl-THF, which is stable. However, even this method still has limitations and does not accurately represent all physiological folate pools in biological samples.

Accordingly, it is an object of the present invention to provide improved methods and tools for measurement of physiological folate pools, in particular methods and tools that can be used to increase the accuracy and/or sensitivity of a subsequent measurement such as MS.

The present invention thus provides a method of stabilising a biological sample comprising at least two folate species, the method comprising the following steps:
- providing the biological sample comprising at least two folate species, and
- contacting the at least two folate species with a reducing agent and an aliphatic aldehyde or ketone under conditions which allow reductive alkylation of at least one (preferably of at least two, especially of all) of the at least two folate species. The reducing agent is a deuterated reducing agent and the aliphatic aldehyde or ketone is a deuterated aliphatic aldehyde or ketone, such that at least one stabilised folate species isotopically labelled with deuterium (D) is obtained (preferably at least one, more preferably at least two, especially all of compounds I-VIII given below is obtained, with or without the ¹³C label, preferably with the ¹³C label). Typically, the reducing agent and/or the aliphatic aldehyde or ketone is fully deuterated (i.e. all H atoms replaced by D atoms).

The present invention also provides a biological sample containing at least one stabilised folate species isotopically labelled with D and preferably with ¹³C, obtainable by the inventive method.

In another aspect, the present invention also provides a biological sample containing at least one (preferably at least two, more preferably at least three, even more preferably at least four, yet even more preferably at least five, especially at least six) stabilised folate species selected from the group consisting of compounds I-VIII: 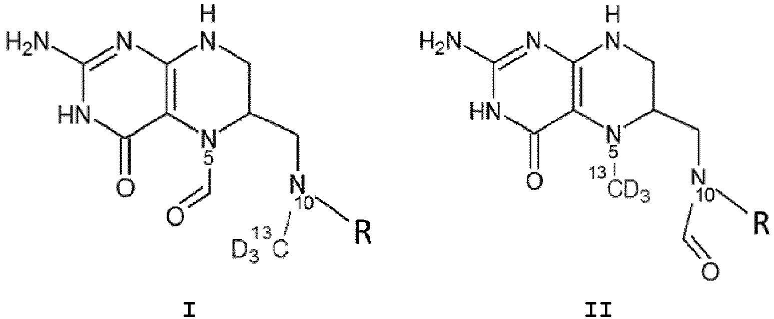 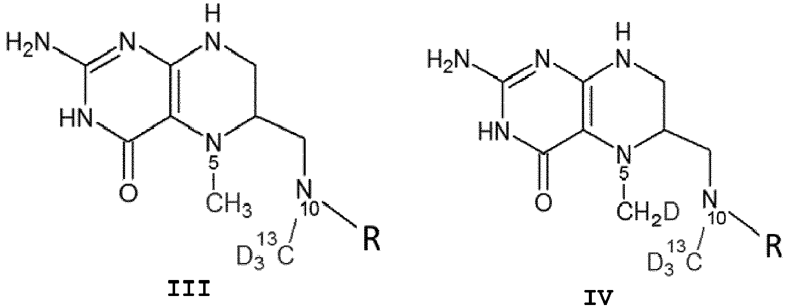 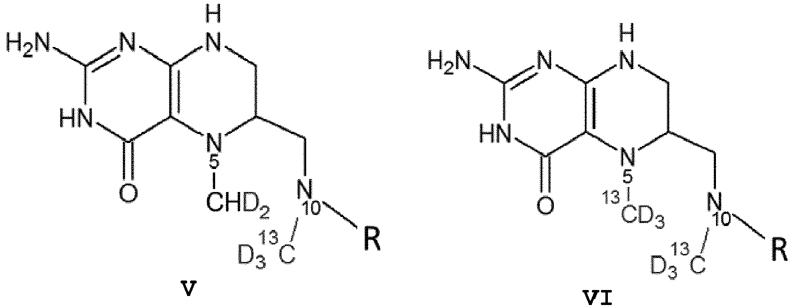 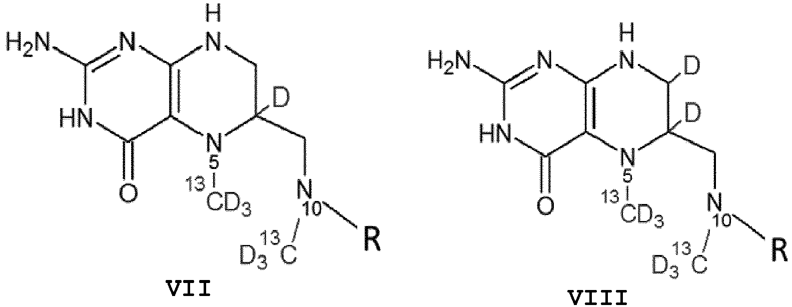 wherein R is 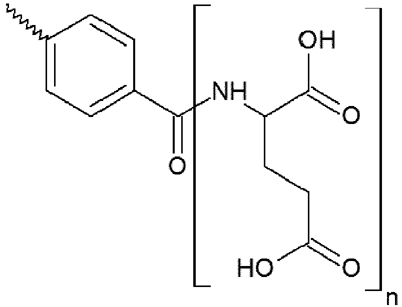 wherein n is an integral from 1 to 8, such as 1, 2, 3, 4, 5, 6, 7 or 8.

The present invention also relates to a standard for mass spectrometry, comprising at least one of compounds I-VIII as defined above, preferably at least two, more preferably at least three, even more preferably at least four, yet even more preferably at least five, especially at least six.

In yet another aspect, the present invention provides a method for quantifying folate species in a biological sample, the method comprising the following steps:
- providing the biological sample of the present invention,
- optionally, purifying the biological sample in a liquid chromatography system, and
- analysing at least a fraction of the biological sample, which fraction contains at least a portion of the at least one stabilised folate species, in a mass spectrometer to measure the abundance of the at least one stabilised folate species.

In yet another aspect, the present invention provides a kit for stabilising a biological sample, which sample comprises at least two folate species, for mass spectrometric analysis, the kit comprising
- a first container containing a reducing agent, wherein the reducing agent is deuterated,
- a second container containing an aliphatic aldehyde or ketone, wherein the aliphatic aldehyde or ketone is deuterated, and
- a third container containing a liquid comprising an organic solvent.

In the course of the present invention, it was surprisingly found that reductive alkylation with isotope-labelled reagents successfully stabilised physiological folate species in a biological sample and - also thereby - allowed for obtaining a more accurate and complete picture of folate pools in biological samples. Further, by employing methods and tools as described above, folate species in a biological sample may be stabilized by derivatization directly in the quenching solution and the information of the oxidation state of both, folate ring and C1 unit, may be encoded as isotopologue derivatives. The stable derivatives may be readily analysed by LC-MS with improved sensitivity compared to non-derivatized folates. Moreover, in typical use cases, the derivatization chemistry is compatible with metabolites up- and downstream of C1 metabolism, allowing a complete depiction of C1 metabolism in a single analysis.

In addition, the greatly simplified sample handling achieved by methods and kits of the present invention is especially beneficial in a clinical setting, where strictly time-controlled sample preparation is often infeasible. Beyond that, determination of folates in food and feed are also improved by relying on the present invention.

The detailed description given below relates to all of the above aspects of the invention unless explicitly excluded.

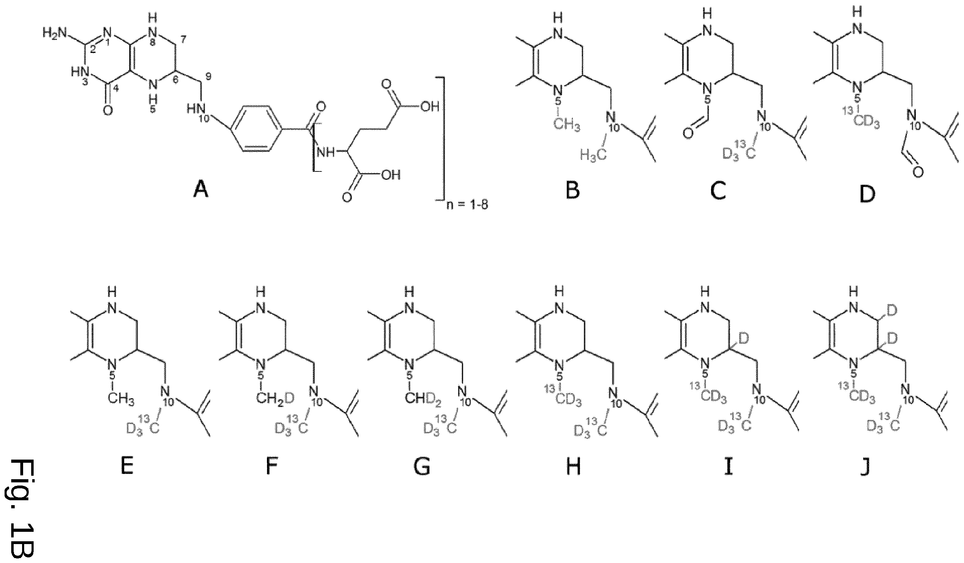

Typically, the pteridine ring of C1-carrying folate species is fully reduced to THF (see Fig. 1B, compound A) and C1 units are either attached to nitrogen 5 (N5), nitrogen 10 (N10) or both forming a bridge between the nitrogen moieties. Oxidation of the pteridine ring is one commonly observed degradation reaction of folates, especially when N5 is unsubstituted. Oxidation may be minimised by adding various antioxidants during extraction of a biological sample. More severely, migration or loss of the C1 group can occur in a pH dependent manner. Folate species carrying C1 units at the oxidation level of formic acid usually exist in a pH dependent equilibrium in protein free form (Jägerstad et al.). It was found that low pH (< 4.5) strongly favours the 5,10-methenyl THF state of this equilibrium, which however is unstable at higher pH (as used in typical sample extraction buffers), leading to conversion to either 5-formyl THF or 10-formyl THF. In the course of the present invention, it was found that, to inhibit interconversion of these three folate species, the free C1 binding site has to be chemically blocked. Choosing appropriate blocking reactions was challenging, as both N5 and N10 are secondary amines and the choice of potential derivatization reactions is limited by the aqueous environment. Surprisingly, it turned out that unoccupied N5 and N10 of folate species can be rapidly and quantitatively protected by reductive alkylation (see examples).

Reductive alkylation (also termed reductive amination, dependent on which compound is considered substrate and which is reagent) is commonly used in amine synthesis. Generally, the reaction involves conversion of the carbonyl group of an aldehyde or ketone reagent to an amine via an intermediate imine in the presence of a reducing agent. A substrate having an amine is alkylated in this process. Reductive alkylation is also used for the derivatisation of proteins, e.g. in crystallography or proteomics (Boersema et al.; Fang et al.; Kovanich et al.; Schittmayer et al.). Consequently, as the reaction is generally known, the skilled person, upon having read the present specification, is able to select conditions which allow reductive alkylation of at least one of the at least two folate species. Temperatures below 25°C, preferably below 20°C, more preferably below 15°C, even more preferably below 10°C, especially below 5°C (but still sufficiently high that the solution in which reductive alkylation is performed remains liquid, e.g. 0°C) are preferred. Furthermore, an acidic pH (e.g. below 5 or 4) is preferred. Using either preferred pH range or preferred temperature (especially both) improve the results in a subsequent MS measurement.

As used herein, "conditions which allow reductive alkylation of at least one of the at least two folate species" typically require that the deuterated reducing agent is (initially) present at a concentration of at least 0.0001 mM, preferably at least 0.001 mM, more preferably at least 0.01 mM, even more preferably at least 0.1 mM, yet even more preferably at least 1 mM or even at least 10 mM. In addition, or alternatively thereto, it is typically required that the deuterated aliphatic aldehyde or ketone is (initially) present at a concentration of at least 0.0001 mM, preferably at least 0.001 mM, more preferably at least 0.01 mM, even more preferably at least 0.1 mM, yet even more preferably at least 1 mM or even at least 10 mM.

As used herein, the term "biological sample" refers to any sample obtained from a biological source (e.g. a living organism such as a mammal), said obtaining optionally comprising processing steps such as drying, freezing, homogenising or fractionating. It is however preferred to avoid such processing steps as much as possible in order to increase accuracy of the folate species measurement. Typically, the biological sample further comprises at least one compound (preferably at least two, more preferably at least three, especially at least four) selected from the group of sugars, amino acids, tricarboxylic acid cycle diacid components (such as oxaloacetic acid, fumaric acid, α-ketoglutaric acid, malic acid, succinic acid), and nucleotides.

Preferably, the biological sample comprises at least three, preferably at least four, more preferably at least five, even more preferably at least six, yet even more preferably at least seven folate species, especially at least three, preferably at least four, more preferably at least five, even more preferably at least six, yet even more preferably at least seven folate species selected from the group consisting of THF, 5-methyl THF, 5,10-methylene THF, 5,10-methenyl THF, 5-formyl THF, 10-formyl THF, DHF and FA.

Specifically, within the context of the present invention, the biological sample is preferably obtained from an individual, preferably a mammal, in particular a human. In particular, the individual has or is suspected of having a disease or condition, in particular selected from the group of cancers, cardiovascular diseases or neural tube defects.

In order to work as closely to the physiological state of folate pools as possible, it is highly preferred that the biological sample comprises cells (such as mammalian or human cells) which comprise the at least two folate species. Preferably, the biological sample is a tissue sample, in particular a biopsy sample.

According to a preferred embodiment, the inventive method further comprises the step of adding a liquid comprising an organic solvent to the biological sample before said contacting step. Preferably, the liquid contains the reducing agent and/or the aliphatic aldehyde or ketone. In addition, or alternatively thereto, it is also preferred when the liquid is able to lyse the cells. The liquid may comprise water. However, it is preferred when the organic solvent content of the liquid is more than 50 %(v/v), preferably more than 60 % (v/v), more preferably more than 70 % (v/v) and/or the water content of the liquid is less than 50 %(v/v), preferably less than 40 %(v/v), more preferably less than 30 %(v/v), even more preferably less than 25 % (v/v).

It is particularly preferred when the liquid is added directly to the cells (i.e. when they are still intact), preferably the tissue sample, in particular the biopsy sample, especially when it contains the reducing agent and the aliphatic aldehyde or ketone. This increases the accuracy of a subsequent MS measurement. Preferably, before said adding step, the cells are concentrated (e.g. by centrifugation) and/or at least a portion of the liquid fraction (e.g. cell medium) of a suspension the cells are in is removed.

According to a further preferred embodiment, the inventive method further comprising the step of lysing the cells, preferably by the liquid and/or in the liquid, especially under conditions which allow reductive alkylation of at least one of the at least two folate species.

To increase the quality of a subsequent measurement by retaining the physiological folate status as much as possible, the inventive method further comprises, in a preferred embodiment, the step of incubating the biological sample at a temperature below 20°C, preferably below 15°C, more preferably below 10°C, even more preferably below 5°C for a period of time before and/or during said contacting step. In addition, or alternatively thereto, the biological sample may be incubated at a pH between 0 and 6.75, preferably between 1 and 6, more preferably between 2 and 5, even more preferably between 3 and 5, yet even more preferably between 4 and 5, in particular between 4.25 and 4.75 for a period of time before, during, and/or after said contacting step. Said period of time may e.g. be between 0.01 and 24 hours, for instance between 5 and 360 minutes. It is preferred when the aforementioned conditions already apply to lysis of the cells of the biological sample, especially when lysis is performed by the liquid.

In the course of the present invention, it was found that labelling the folate species with ¹³C improved the accuracy of measurement in MS. Therefore, the aliphatic aldehyde or ketone preferably has a ¹³C carbon atom (in particular carbonyl carbon atom), such that the at least one stabilised folate species is obtained further isotopically labelled with ¹³C.

According to an especially preferred embodiment, the aliphatic aldehyde or ketone is deuterated formaldehyde, deuterated acetaldehyde, deuterated propionaldehyde or deuterated butyraldehyde.

Formaldehyde-¹³C, D₂ was found to be particularly beneficial for obtaining high-quality measurement results in the course of the present invention and is hence a particularly preferred aliphatic aldehyde or ketone for use in the present invention.

It has turned out that cyanoborohydride is particularly suitable for reductive alkylation of folate species. Accordingly, deuterated cyanoborohydride (i.e. cyanoborodeuteride), such as sodium cyanoborodeuteride (NaBD₃CN), is highly preferred as reducing agent in methods and kits of the present invention.

In the course of the present invention, the following parameters of the liquid have been found to be of particular importance, in particular for good performance of the folate alkylation reaction and subsequent processing steps: dielectric constant, solubility of the reducing agent and/or the aliphatic aldehyde or ketone in the liquid, pH and volatility of the liquid.

Thus, according to a preferred embodiment, the liquid has a dielectric constant of more than 15, preferably more than 20, more preferably more than 25, especially more than 27.5, at a temperature of 20°C.

In a further preference, the reducing agent, preferably cyanoborodeuteride, has a solubility in the liquid of more than 10 g/L, preferably more than 50 g/L, more preferably more than 100 g/L, even more preferably more than 250 g/L, yet even more preferably more than 500 g/L, especially more than 750 g/L or even more than 1000 g/L at a temperature of 20°C. Alternatively, or in addition thereto, the aliphatic aldehyde or keton, preferably formaldehyde, has a solubility in the liquid of more than 10 g/L, preferably more than 50 g/L, more preferably more than 100 g/L, even more preferably more than 250 g/L, yet even more preferably more than 500 g/L, especially more than 750 g/L or even more than 1000 g/L at a temperature of 20°C.

According to particular preference, the liquid further comprises an acid, preferably an organic acid, and has a pH between 0 and 6.75, preferably between 1 and 6, more preferably between 2 and 5, even more preferably between 3 and 5, yet even more preferably between 4 and 5, in particular between 4.25 and 4.75. Such organic acid may be e.g. such as acetic acid, formic acid or propionic acid or a mixture thereof.

According to a further preferred embodiment, the liquid has a boiling point of less than 100°C, preferably less than 90°C, more preferably less than 80°C, especially less than 70°C or even less than 65°C, at atmospheric pressure.

Methanol and acetonitrile were found to be highly suitable for the purposes of the present invention (in particular for achieving good performance of the reductive folate alkylation). Accordingly, the organic solvent is preferably methanol, acetonitrile or a mixture thereof.

It is beneficial when the volume concentration of the organic solvent in the liquid is at least 40%, preferably at least 50%, more preferably at least 60%, even more preferably at least 70%, especially at least 75. Preferably, the liquid further comprises water at a volume concentration of least 1%, preferably at least 5%, more preferably at least 10%.

In a further preferred embodiment of the present invention, the method further comprises the step of concentrating and/or drying the biological sample, or a fraction thereof which fraction contains the at least one stabilised folate species. Any concentrating or drying method known in the art may be used, for instance rotary evaporation or lyophilisation.

The biological sample of the present invention is preferably dry (after the inventive stabilisation method has been applied), as this further increases stability. It is evident that the presence of a certain level of residual moisture in the biological sample of the present invention is not excluded by the expression "dry", for the presence of a detectable low level of residual moisture can typically not be avoided completely. Preferably, the residual moisture of the inventive biological sample does not exceed 10% (w/w), in particular it does not exceed 5% (w/w), or even does not exceed 1% (w/w).

Typically, the stabilised folate species is present in the sample at a concentration of at least 0.0001 ppm, preferably at least 0.001 ppm, more preferably at least 0.01 ppm, even more preferably at least 0.1 ppm, in particular at least 1 ppm or even at least 10 ppm. Herein, unless specified otherwise, "ppm" is to be understood as "ppmw" (i.e. parts-per-million by weight) .

For optional LC-MS analysis of the biological sample of the present invention, any suitable LC-MS known in the art may be used.

In respect the LC step, good signal to noise ratios were achieved with hydrophilic interaction liquid chromatography, in particular with a polymer-based zwitterionic stationary phase. Consequently, in a preferred embodiment of the inventive method for quantifying folate species in a biological sample, the purifying step comprises hydrophilic interaction liquid chromatography, in particular with a polymer-based zwitterionic stationary phase.

In respect to the MS step, analysis by multiple-reaction monitoring (MRM) turned out to be very suitable. Accordingly, in a preferred embodiment of the inventive method for quantifying folate species in a biological sample, the mass spectrometer is preferably operated in an MRM mode.

Turning to the inventive kit for stabilising a biological sample, the kit's reducing agent, aliphatic aldehyde or ketone and liquid are preferably as defined hereinabove. Moreover, it is highly preferred that the liquid further contains an acid (e.g. an organic acid as defined above) and/or an acid (e.g. an organic acid as defined above) is provided in a fourth container. For instance, the first container may contain the reducing agent in dissolved form, e.g. in a solution such as 1M NaOH, and/or the second container may contain the aliphatic aldehyde or ketone dissolved in water and/or an organic solvent.

The kit preferably further comprises at least one container containing at least one of compounds I-VIII as defined above, preferably for use as a control in MS, or the standard for MS of the present invention. The kit may further comprise usage instructions, e.g. instructions to use the kit according to the inventive method. The kit may further comprise another container with a, preferably dry, biological sample of the present invention as a reference sample.

Preferably, the container containing at least one of compounds I-VIII as defined above, preferably for use as a control in mass spectrometry, or the inventive standard for mass spectroscopy contains at least two, preferably at least two, more preferably at least three, even more preferably at least four, yet even more preferably at least five, especially at least six of said compounds.

In a particularly preferred embodiment of the inventive standard for MS, a solid preparation such as a powder essentially consisting of at least one of compounds I-VIII as defined above or a salt thereof, or a solution essentially consisting of at least one of compounds I-VIII dissolved in a, preferably organic, solvent is provided. In this connection, the expression "essentially consisting" preferably relates to a concentration of contaminants in the solid preparation or solution that is lower than 10% (w/w), preferably lower than 5% (w/w), more preferably lower than 2.5% (w/w), even more preferably lower than 1% (w/w), yet even more preferably lower than 0.5% (w/w), especially lower than 0.1% (w/w) or even lower than 0.01% (w/w).

The present invention further relates to the following embodiments:
Embodiment 1. A method of stabilising a biological sample comprising at least two folate species, the method comprising the following steps:
   - providing the biological sample comprising at least two folate species, and
   - contacting the at least two folate species with a reducing agent and an aliphatic aldehyde or ketone under conditions which allow reductive alkylation of at least one of the at least two folate species, wherein the reducing agent is a deuterated reducing agent and the aliphatic aldehyde or ketone is a deuterated aliphatic aldehyde or ketone, whereby at least one stabilised folate species isotopically labelled with D is obtained.
Embodiment 2. The method of embodiment 1, wherein the aliphatic aldehyde or ketone has a 13C carbon atom, wherein the at least one stabilised folate species is obtained further isotopically labelled with 13C.
Embodiment 3. The method of embodiment 1 or 2, wherein the reducing agent is a cyanoborodeuteride.
Embodiment 4. The method of any one of embodiments 1 to 3, wherein the aliphatic aldehyde or ketone is deuterated formaldehyde, acetaldehyde, propionaldehyde or butyraldehyde.
Embodiment 5. The method of embodiment 4, wherein the aliphatic aldehyde or ketone is formaldehyde-13C,D2.
Embodiment 6. The method of any one of embodiments 1 to 5, wherein the biological sample comprises cells which comprise the at least two folate species, preferably wherein the biological sample is a tissue sample, in particular a biopsy sample.
Embodiment 7. The method of any one of embodiments 1 to 6, further comprising the step of adding a liquid comprising an organic solvent to the biological sample before said contacting step, preferably wherein the liquid contains the reducing agent and the aliphatic aldehyde or ketone.
Embodiment 8. The method of embodiment 7, wherein the liquid has a dielectric constant of more than 15, preferably more than 20, more preferably more than 25, especially more than 27.5, at a temperature of 20°C and/or wherein the liquid has a boiling point of less than 100°C, preferably less than 90°C, more preferably less than 80°C, especially less than 70°C or even less than 65°C, at atmospheric pressure.
Embodiment 9. The method of embodiment 7 or 8, wherein the organic solvent is methanol, acetonitrile or a mixture thereof.
Embodiment 10. The method of any one of embodiments 7 to 9, wherein the volume concentration of the organic solvent in the liquid is at least 40%, preferably at least 50%, more preferably at least 60%, even more preferably at least 70%, especially at least 75%; preferably wherein the liquid further comprises water preferably at a volume concentration of least 1%, preferably at least 5%, more preferably at least 10%.
Embodiment 11. The method of any one of embodiments 7 to 10, wherein the reducing agent, preferably cyanoborodeuteride, has a solubility in the liquid of more than 10 g/L, preferably more than 50 g/L, more preferably more than 100 g/L, even more preferably more than 250 g/L, yet even more preferably more than 500 g/L, especially more than 750 g/L or even more than 1000 g/L at a temperature of 20°C, and/or the aliphatic aldehyde or ketone, preferably formaldehyde, has a solubility in the liquid of more than 10 g/L, preferably more than 50 g/L, more preferably more than 100 g/L, even more preferably more than 250 g/L, yet even more preferably more than 500 g/L, especially more than 750 g/L or even more than 1000 g/L at a temperature of 20°C.
Embodiment 12. The method of any one of embodiments 7 to 11, wherein the liquid further comprises an acid, preferably an organic acid such as acetic acid, formic acid or propionic acid or a mixture thereof, and has a pH between 0 and 6.75, preferably between 1 and 6, more preferably between 2 and 5, even more preferably between 3 and 5, yet even more preferably between 4 and 5, in particular between 4.25 and 4.75.
Embodiment 13. The method of any one of embodiments 7 to 12, wherein the liquid is added directly to the cells, preferably the tissue sample, in particular the biopsy sample.
Embodiment 14. The method of any one of embodiments 6 to 13, further comprising the step of lysing the cells, preferably by the liquid.
Embodiment 15. The method of any one of embodiments 1 to 14, further comprising incubating the biological sample at a temperature below 20°C, preferably below 15°C, more preferably below 10°C, even more preferably below 5°C for a period of time before and/or during said contacting step, and/or at a pH between 0 and 6.75, preferably between 1 and 6, more preferably between 2 and 5, even more preferably between 3 and 5, yet even more preferably between 4 and 5, in particular between 4.25 and 4.75 before, during, and/or after said contacting step.
Embodiment 16. The method of any one of embodiments 1 to 15, further comprising the step of concentrating and/or drying the sample, or a fraction thereof, containing the at least one stabilised folate species.
Embodiment 17. A biological sample containing at least one stabilised folate species isotopically labelled with D and preferably with 13C, obtainable by the method of any one of embodiments 1 to 16; preferably wherein the biological sample further comprises at least one compound (preferably at least two, more preferably at least three, especially at least four) selected from the group of sugars, amino acids, tricarboxylic acid cycle diacid components (such as oxaloacetic acid, fumaric acid, α-ketoglutaric acid, malic acid, succinic acid), and nucleotides.
Embodiment 18. A biological sample containing at least one stabilised folate species selected from the group consisting of compounds I-VIII: 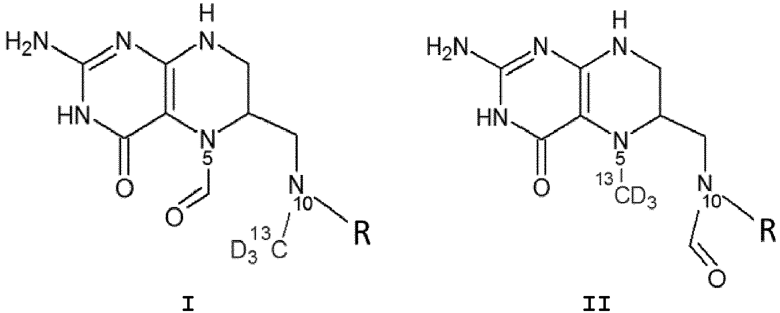 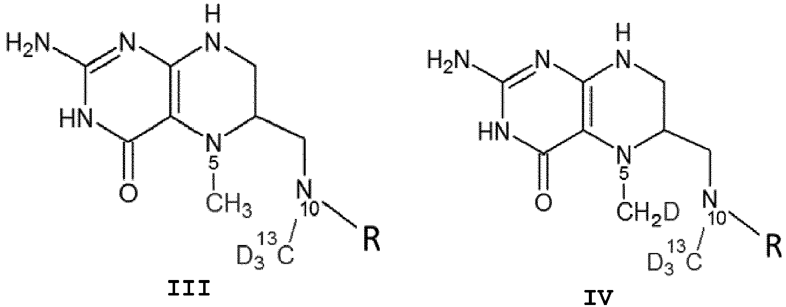 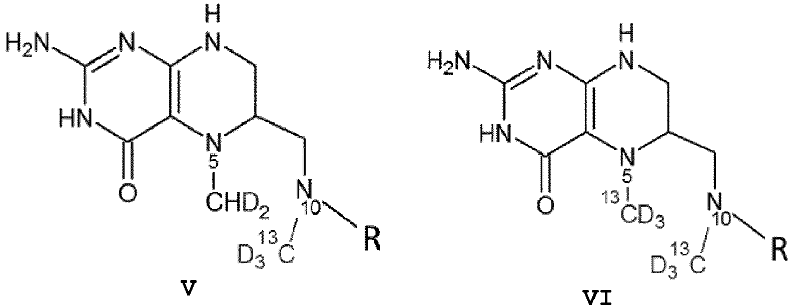 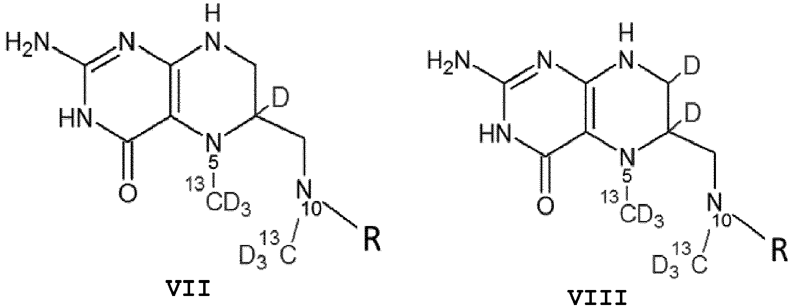 wherein R is 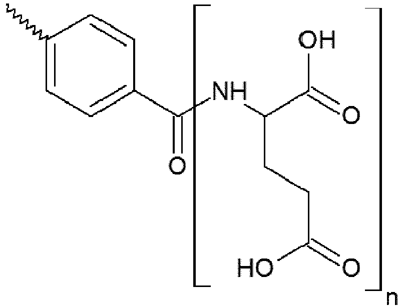 wherein n is an integral from 1 to 8;
   preferably wherein the biological sample further comprises at least one compound (preferably at least two, more preferably at least three, especially at least four) selected from the group of sugars, amino acids, tricarboxylic acid cycle diacid components (such as oxaloacetic acid, fumaric acid, α-ketoglutaric acid, malic acid, succinic acid), and nucleotides.
Embodiment 19. The biological sample of embodiment 17 or 18, wherein the sample is dry.
Embodiment 20. The biological sample of any one of embodiments 17 to 19, wherein the stabilised folate species is present in the sample at a concentration of at least 0.0001 ppm, preferably at least 0.001 ppm, more preferably at least 0.01 ppm, even more preferably at least 0.1 ppm, in particular at least 1 ppm or even at least 10 ppm.
Embodiment 21. A method for quantifying folate species in a biological sample, the method comprising the following steps:
   - providing a biological sample as defined in any one of embodiments 17 to 20,
   - optionally, purifying the biological sample in a liquid chromatography system, and
   - analysing at least a fraction of the biological sample, which fraction contains at least a portion of the at least one stabilised folate species, in a mass spectrometer to measure the abundance of the at least one stabilised folate species.
Embodiment 22. A kit for stabilising a biological sample comprising at least two folate species for mass spectrometric analysis, the kit comprising
   - a first container containing a reducing agent, wherein the reducing agent is deuterated,
   - a second container containing an aliphatic aldehyde or ketone, wherein the aliphatic aldehyde or ketone is deuterated, and
   - a third container containing a liquid comprising an organic solvent;
   wherein the liquid further contains an acid and/or an acid is provided in a fourth container.
Embodiment 23. The kit of embodiment 22, wherein the aliphatic aldehyde or ketone has a 13C carbon atom.
Embodiment 24. The kit of embodiment 22 or 23, wherein the reducing agent is a cyanoborodeuteride.
Embodiment 25. The kit of any one of embodiments 22 to 24, wherein the aliphatic aldehyde or ketone is deuterated formaldehyde, acetaldehyde, propionaldehyde or butyraldehyde.
Embodiment 26. The kit of embodiment 25, wherein the aliphatic aldehyde or ketone is formaldehyde-13C,D2.
Embodiment 27. The kit of any one of embodiments 22 to 26, wherein the liquid has a dielectric constant of more than 15, preferably more than 20, more preferably more than 25, especially more than 27.5, at a temperature of 20°C and/or wherein the liquid has a boiling point of less than 100°C, preferably less than 90°C, more preferably less than 80°C, especially less than 70°C or even less than 65°C, at atmospheric pressure.
Embodiment 28. The kit of any one of embodiments 22 to 27, wherein the organic solvent is methanol, acetonitrile or a mixture thereof.
Embodiment 29. The kit of any one of embodiments 22 to 28, wherein the volume concentration of the organic solvent in the liquid is at least 40%, preferably at least 50%, more preferably at least 60%, even more preferably at least 70%, especially at least 75%; preferably wherein the liquid further comprises water preferably at a volume concentration of least 1%, preferably at least 5%, more preferably at least 10%.
Embodiment 30. The kit of any one of embodiments 22 to 29, wherein the reducing agent, preferably cyanoborodeuteride, has a solubility in the liquid of more than 10 g/L, preferably more than 50 g/L, more preferably more than 100 g/L, even more preferably more than 250 g/L, yet even more preferably more than 500 g/L, especially more than 750 g/L or even more than 1000 g/L at a temperature of 20°C, and/or the aliphatic aldehyde or keton, preferably formaldehyde, has a solubility in the liquid of more than 10 g/L, preferably more than 50 g/L, more preferably more than 100 g/L, even more preferably more than 250 g/L, yet even more preferably more than 500 g/L, especially more than 750 g/L or even more than 1000 g/L at a temperature of 20°C.
Embodiment 31. The kit of any one of embodiments 22 to 30, wherein the acid is an organic acid such as acetic acid, formic acid or propionic acid or a mixture thereof.
Embodiment 32. The kit of any one of embodiments 22 to 31, wherein the liquid has a pH between 0 and 6.75, preferably between 1 and 6, more preferably between 2 and 5, even more preferably between 3 and 5, yet even more preferably between 4 and 5, in particular between 4.25 and 4.75.
Embodiment 33. The kit of any one of embodiments 22 to 32, further comprising at least one container containing at least one of compounds I-VIII as defined in embodiment 18, preferably for use as a control in mass spectrometry.
Embodiment 34. The kit of any one of embodiments 22 to 33, further comprising usage instructions.
Embodiment 35. A standard for mass spectrometry, comprising at least one of compounds I-VIII as defined in embodiment 18.

The present invention is further illustrated by the following figures and examples, without being restricted thereto.
**Fig. 1A****:** One-carbon cycle showing the connection between folate metabolism and methionine cycle (adapted from Iacobazzi et al.). Color coding: purple: C1 donors; green: folates; blue: products of C1 metabolism and downstream pathways; white: not covered by method. Abbreviations: CTH, cystathionine γ-lyase; CBS, cystathionine β-synthase; DHF, dihydrofolate; FA, folic acid; Hcy, homocysteine; MAT, methionine adenosyltransferase; MS, methionine synthase; MTHFR, methylenetetrahydrofolate reductase; 5-MTHF, 5-methyl-tetrahydrofolate; MT, methyltransferase; SAH, S-adenosylhomocysteine; SAHH, SAH hydrolase; SAM, S-adenosylmethionine; SHMT, serine hydroxymethyltransferase; THF, tetrahydrofolate; B6, vitamin B6; B12, vitamin B12.
**Fig. 1B****:** (A) THF; (B) Isotopologue synthesized with unlabelled reagents, providing an isotopic standard; (C-J) Stabilised folate species, groups and atoms introduced by reductive alkylation with cyanoborodeuteride and formaldehyde-¹³C, D₂ are shown in red (cf. compounds I-VIII as disclosed herein). The stabilised folate species can be readily analysed by LC-MS with improved sensitivity compared to non-derivatized folates. Depictions of compounds C-J are cut at the right-hand side, as their remaining structure is unchanged from compound A.
**Fig. 2****:** Crosstalk between MS measurement channels; Purple: calculated; Green: measured. No cross-talk between adjacent channels exceeding the expected effect from natural isotope abundances was observed.
**Fig. 3****:** Stability of folate species at 4°C after stabilisation by reductive alkylation with cyanoborodeuteride and formaldehyde-¹³C, D₂ (original folate species names used). The stabilised folate species showed excellent stability. To avoid any effects caused by further sample preparation, no steps to remove excess sodium cyanoborodeuteride were carried out. The resulting hydrogen bubble formation in the sample vial explains the fluctuations at the beginning of the stability experiment.
**Fig. 4****:** Stability of folate species after stabilisation by reductive alkylation with cyanoborodeuteride and formaldehyde-¹³C, D₂, before and after rotary vacuum concentration (original folate species names used). Relative abundance of folate species in an *E. coli* extract stabilised according to the present invention remained unchanged pre- and post-concentration
**Fig. 5****:** Linearity of MS quantification after stabilisation by reductive alkylation with cyanoborodeuteride and formaldehyde-¹³C, D₂ (original folate species names used).
**Fig. 6****:** Matrix effects in MS measurement after stabilisation by reductive alkylation.
**Fig. 7A****:** Quantification results of trimethoprim inhibition experiment with *E. coli* using the inventive sample stabilisation method (original folate species names used). Purple: Control; Green: Trimethoprim-treated.
**Fig. 7B****:** Trimethoprim inhibition experiment, domino effect observed (original folate species names used). Purple: Control; Green: Trimethoprim-treated.
**Fig. 7C****:** Trimethoprim inhibition experiment, up- and downstream metabolites. Evidently, the method of the present invention is compatible with measurement of metabolites up- and downstream of C1 metabolism, allowing a complete depiction of C1 metabolism in a single analysis. Purple: Control; Green: Trimethoprim-treated.
**Fig. 8A****:** Quantification results of methotrexate inhibition experiment in HepG2 cells using the inventive sample stabilisation method (original folate species names used). Purple: Control; Green: Methotrexate-treated.
**Fig. 8B****:** Methotrexate inhibition experiment, up- and downstream metabolites. Evidently, the method of the present invention is compatible with measurement of metabolites up- and downstream of C1 metabolism, allowing a complete depiction of C1 metabolism in a single analysis. Purple: Control; Green: Methotrexate-treated.

### Examples

### Example 1 - Chemical stabilisation of folate species by reductive alkylation with isotopic encoding

Standards of 5-methyl THF, 5,10-methylen THF, 5,10-methenyl THF, THF, DHF, FA and 5-formyl THF were purchased from Schircks Laboratories, Bauma, Switzerland. 10-formyl THF was synthesized from 5-formyl THF as published by Stover et al.

Folate standards were dissolved in ice cold 80 % methanol, containing 30 mM NaCNBD₃, 0.2 % Formaldehyde-13C, D₂ (∼67 mM) and 0.1 % acetic acid to a concentration of 0.1 mg/mL for chemical stabilisation. Samples were incubated for 60 min on ice and stored at -20 °C until measured.

In the chemical stabilisation reaction, 5-formyl THF was methylated at N10 yielding the 5-formyl, 10-methyl THF (Fig. 1B, compound C) and 10-formyl THF yielded the N5-methyl, N10-formyl derivative (Fig. 1B, compound D). These two structural isomers could be separated by chromatography and could additionally be discriminated by MS2. Employing heavy isotope labelled reagents (i.e. NaCNBD₃, formaldehyde-¹³C, D₂) allowed to discriminate between native and synthetic C1 groups of dimethylated species, so derivatized 5-methyl THF (Fig. 1B, compound E) could be distinguished from derivatized THF (Fig. 1B, compound H) by mass spectrometry. Furthermore, since bridged species were reduced by NaCNBD₃, the initial oxidation state of the native C1 unit was encoded in the isotopic composition of the derivative, with - higher oxidation states incorporating more deuterium (Fig. 1B, compounds F and G). Finally, the pteridine ring of folic acid was also readily reduced by NaCNBD₃ and subsequently methylated at N5. Dependent on the initial oxidation state of the pteridine ring either one (Fig. 1B, compound I) or two deuterium (Fig. 1B, compound J) were introduced in the pteridine ring.

Taken together, stabilisation resulted in structurally different and chemically stable analytes, greatly simplifying analysis and sample handling. Six of the initial folate species were encoded as isotopologues and a seventh isotopologue could easily be synthesized with unlabelled reagents, providing an isotopic standard (Fig. 1A, compound B) for derivatives of 5-methyl THF, 5,10-methylen THF and 5,10-methenyl THF, DHF, THF, and FA.

### Example 2 - LC-MS method

Based on the highly hydrophilic character of both poly-glutamated folates and the derivatives, either ion pairing chromatography or hydrophilic interaction liquid chromatography (HILIC) are potential methods for separation of folates before MS analysis (Lu et al.; Freisleben et al.). Two HILIC solid phases were tested, namely the aminopropyl modified silica employed by Lu et al. (Luna-NH2, Phenomenex) and a polymer based zwitterionic stationary phase (ZIC-pHILIC, Merck). The latter yielded lower noise levels and accompanying better signal to noise ratios. Given the low abundance of the individual folate species and the required sensitivity, a targeted method employing an ABSciex 5500 QTrap in MRM mode was used. As a total of 72 folate species plus several up and downstream metabolites was to be measured, a scheduled MRM method with windows of 60 seconds per analyte and target scan time of 1.5 s was used (see details below).

The following method was used in all MS experiments unless stated otherwise:
Liquid chromatography was carried out in hydrophilic interaction chromatography mode on an Agilent Technologies Infinity 1290 UHPLC system. The column was a SeQuant ZIC-pHILIC 150 x 2.1 mm plus guard column 20 x 2.1 mm (Merck, Darmstadt, Germany). The solvents were acetonitrile without any further additives (solvent A) and 20 mM (NH₄)₂CO₃ in H₂O, adjusted to pH = 9.2 with ammonium hydroxide solution (solvent B). The following gradient was run at a constant flow rate of 100 µL/min: 0 min, 30 % B; 2 min, 30 % B; 18 min, 70 % B; 20 min, 95 % B; 23 min, 95% B; followed by re-equilibration at 30 % B for 7 min. The column compartment temperature was 30 °C.

Mass spectrometry was performed on an ABSciex 5500 QTrap mass spectrometer in positive, scheduled MRM mode. The detection window was set to 60 s and the target scan time was 1.5 s. Source parameters were curtain gas: 20 psi; collision gas: medium; ion spray voltage: 5500 V; temperature: 700 °C; ion source gas 1: 40 psi; ion source gas 2: 50 psi. The complete list of transitions and parameters can be found in Table 1 below. Both systems were controlled by Analyst 1.6.2 (ABSciex).

**Table 1:**

| **Q1 Mass (Da)** | **Q2 Mass (Da)** | **Time (min)** | **DP (volts)** | **CE (volts)** |
|---|---|---|---|---|
| 478.232 | 331.179 | 7.0 | 110 | 35 |
| 479.238 | 332.185 | 7.0 | 110 | 35 |
| 480.244 | 333.192 | 7.0 | 110 | 35 |
| 482.254 | 335.201 | 7.0 | 110 | 35 |
| 483.260 | 336.208 | 7.0 | 110 | 35 |
| 484.267 | 337.214 | 7.0 | 110 | 35 |
| 492.211 | 345.158 | 7.9 | 110 | 35 |
| 492.211 | 317.163 | 7.3 | 110 | 35 |
| 494.249 | 347.196 | 7.1 | 110 | 35 |
| 474.210 | 327.157 | 7.9 | 110 | 35 |
| 607.274 | 331.179 | 9.4 | 110 | 44 |
| 608.281 | 332.185 | 9.4 | 110 | 44 |
| 609.287 | 333.192 | 9.4 | 110 | 44 |
| 611.297 | 335.201 | 9.4 | 110 | 44 |
| 612.303 | 336.208 | 9.4 | 110 | 44 |
| 613.309 | 337.214 | 9.4 | 110 | 44 |
| 621.254 | 345.158 | 10.2 | 110 | 44 |
| 621.254 | 317.163 | 9.8 | 110 | 44 |
| 623.292 | 347.196 | 9.5 | 110 | 44 |
| 736.317 | 331.179 | 11.1 | 110 | 54 |
| 737.323 | 332.185 | 11.1 | 110 | 54 |
| 738.330 | 333.192 | 11.1 | 110 | 54 |
| 740.339 | 335.201 | 11.1 | 110 | 54 |
| 741.345 | 336.208 | 11.1 | 110 | 54 |
| 742.352 | 337.214 | 11.1 | 110 | 54 |
| 750.296 | 345.158 | 11.9 | 110 | 53 |
| 750.296 | 317.163 | 11.4 | 110 | 53 |
| 752.334 | 347.196 | 11.2 | 110 | 53 |
| 865.360 | 331.179 | 12.3 | 110 | 63 |
| 866.366 | 332.185 | 12.3 | 110 | 63 |
| 867.372 | 333.192 | 12.3 | 110 | 63 |
| 869.382 | 335.201 | 12.3 | 110 | 63 |
| 870.388 | 336.208 | 12.3 | 110 | 63 |
| 871.394 | 337.214 | 12.3 | 110 | 63 |
| 879.339 | 345.158 | 13.0 | 110 | 63 |
| 879.339 | 317.163 | 12.5 | 110 | 63 |
| 881.377 | 347.196 | 12.4 | 110 | 62 |
| 994.402 | 331.179 | 13.1 | 110 | 73 |
| 995.408 | 332.185 | 13.1 | 110 | 73 |
| 996.415 | 333.192 | 13.1 | 110 | 73 |
| 998.424 | 335.201 | 13.1 | 110 | 72 |
| 999.431 | 336.208 | 13.1 | 110 | 72 |
| 1000.437 | 337.214 | 13.1 | 110 | 72 |
| 1008.381 | 345.158 | 13.7 | 110 | 72 |
| 1008.381 | 317.163 | 13.3 | 110 | 72 |
| 1010.419 | 347.196 | 13.2 | 110 | 72 |
| 1123.445 | 331.179 | 13.8 | 110 | 82 |
| 1124.451 | 332.185 | 13.8 | 110 | 82 |
| 1125.457 | 333.192 | 13.8 | 110 | 82 |
| 1127.467 | 335.201 | 13.8 | 110 | 82 |
| 1128.473 | 336.208 | 13.8 | 110 | 82 |
| 1129.479 | 337.214 | 13.8 | 110 | 82 |
| 1137.424 | 345.158 | 14.4 | 110 | 81 |
| 1137.424 | 317.163 | 14.0 | 110 | 81 |
| 1139.462 | 347.196 | 13.9 | 110 | 81 |
| 627.251 | 331.179 | 14.4 | 110 | 70 |
| 627.754 | 332.185 | 14.4 | 110 | 70 |
| 628.761 | 333.192 | 14.4 | 110 | 70 |
| 634.758 | 335.201 | 14.4 | 110 | 70 |
| 635.763 | 336.208 | 14.4 | 110 | 70 |
| 636.766 | 337.214 | 14.4 | 110 | 70 |
| 641.734 | 345.158 | 14.9 | 110 | 70 |
| 641.237 | 317.163 | 14.5 | 110 | 70 |
| 643.755 | 347.196 | 14.4 | 110 | 70 |
| 691.772 | 331.179 | 14.9 | 110 | 70 |
| 692.276 | 332.185 | 14.9 | 110 | 70 |
| 693.282 | 333.192 | 14.9 | 110 | 70 |
| 699.280 | 335.201 | 14.9 | 110 | 70 |
| 700.285 | 336.208 | 14.9 | 110 | 70 |
| 701.288 | 337.214 | 14.9 | 110 | 70 |
| 706.256 | 345.158 | 15.4 | 110 | 70 |
| 705.758 | 317.163 | 15.0 | 110 | 70 |
| 708.277 | 347.196 | 14.9 | 110 | 70 |
| 112.115 | 66.110 | 6.5 | 50 | 30 |
| 142.126 | 96.121 | 6.9 | 50 | 25 |
| 435.221 | 250.093 | 8.4 | 30 | 35 |
| 421.205 | 170.300 | 5.3 | 35 | 25 |
| 222.092 | 176.087 | 9.5 | 30 | 30 |
| 323.064 | 80.973 | 7.5 | 30 | 30 |
| 349.054 | 137.046 | 10.1 | 60 | 20 |
| 339.070 | 127.061 | 9.7 | 50 | 20 |
| 187.000 | 98.980 | 11.4 | 30 | 25 |
| 664.120 | 524.058 | 8.5 | 60 | 30 |
| 666.130 | 649.106 | 7.8 | 60 | 30 |
| 744.083 | 604.025 | 11.4 | 60 | 30 |
| 746.098 | 729.072 | 11.5 | 60 | 30 |
| 108.093 | 62.088 | 6.6 | 50 | 30 |
| 104.071 | 58.066 | 6.6 | 50 | 30 |
| 118.086 | 58.066 | 6.0 | 50 | 35 |
| 104.107 | 60.081 | 11.6 | 50 | 35 |
| 186.134 | 133.032 | 4.6 | 30 | 20 |
| 172.118 | 126.122 | 5.2 | 30 | 20 |
| 158.103 | 58.995 | 5.7 | 60 | 30 |
| 344.167 | 166.126 | 6.4 | 30 | 30 |
| 685.311 | 166.126 | 9.4 | 30 | 45 |
| 295.226 | 170.103 | 6.9 | 30 | 30 |

A potential source of C1 units is the histidine degradation product 5-formimino THF. Unfortunately, no standard for 5-formimino THF is commercially available. A screen for the predicted derivatization product was conducted *in E. coli* extracts using a product ion scan method. Two compounds corresponding to the triple and quadruple glutamated species were identified. The retention times were consistent with other triple and four-fold glutamated folates and the major MS2 fragment fits the predicted heavy labelled, de-glutamated 5-formimino THF. Based on this data transitions for all 5-formimino THF glutamate species were included into the MRM (cf. Table 1 above).

### Example 3 - Testing completeness and selectivity of the stabilisation reaction

In general, the LC-MS method of Example 2 was used for this example, with some adaptations as disclosed below:
Completeness of the stabilisation reaction according to Example 1 was assessed by running LC-MS analysis in product ion scan mode (including m/z range of unfragmented parent ions) for underivatized, single methylated, doubly methylated and triple (i.e. over-) methylated compounds. The sum of fragment ion signals for underivatized folates and side products were below 2 % of the expected product for all compounds. Among the side products, triple methylated species were the most abundant.

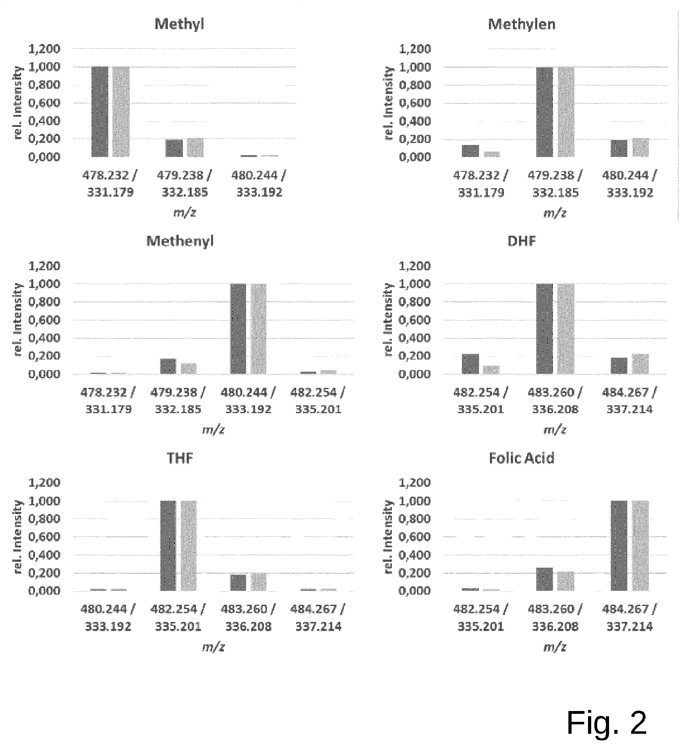

Due to the limited resolution of quadrupole technology, the minimal mass difference of 1 Da in some parent and product masses is a potential source of experimental error. Potential cross-talk between channels at different quadrupole resolution settings was therefore carefully examined using separate standards. At unit quadrupole resolution and higher, no cross-talk between adjacent channels exceeding the expected effect from natural isotope abundances was observed (see Fig. 2).

Since the enrichment of heavy isotopes in labelling reagents is typically not complete (96-98 % according to vendor), also M-1 and M-2 species were considered. The true enrichment of heavy isotopes was slightly higher as specified, as the measured cross-talk was slightly lower for M-1 and M-2 species than the calculated threshold. Accordingly, measured values were used for subsequent deconvolution of signals.

### Example 4 - Testing the stability of the stabilised folate species

Stability of the folate species stabilised according to Example 1 was compared to that of native folate species.

Native folate species were dissolved in 50:50 methanol:water, 0.1% ascorbic acid, 20 mM ammonium acetate, pH = 6.2, according to the method disclosed in Lu et al. After mixing, they were incubated at 4 °C in the auto sampler and injected repeatedly over a time span of 96h. The addition of ascorbic acid effectively slowed oxidation of THF and to some extent also DHF. However, at the near neutral pH, 5,10-methenyl THF degraded with a half life of less than 3 h to 5- or 10-formyl THF (undistinguishable in this prior art method). Moreover, 5,10-methenyl THF was highly unstable under the employed chromatographic conditions employed (pH 9.2, 30 °C) and the signal at the retention time of 10 min was only 11 % (AUC) of that obtained by flow injection. Another unstable folate is the 5,10-methylen folate, which was reported to dissociate rapidly to formaldehyde and THF in the absence of excess formaldehyde at non-alkaline pH (Strandler et al.). The half life of this compound turned out to be even below 30 min.

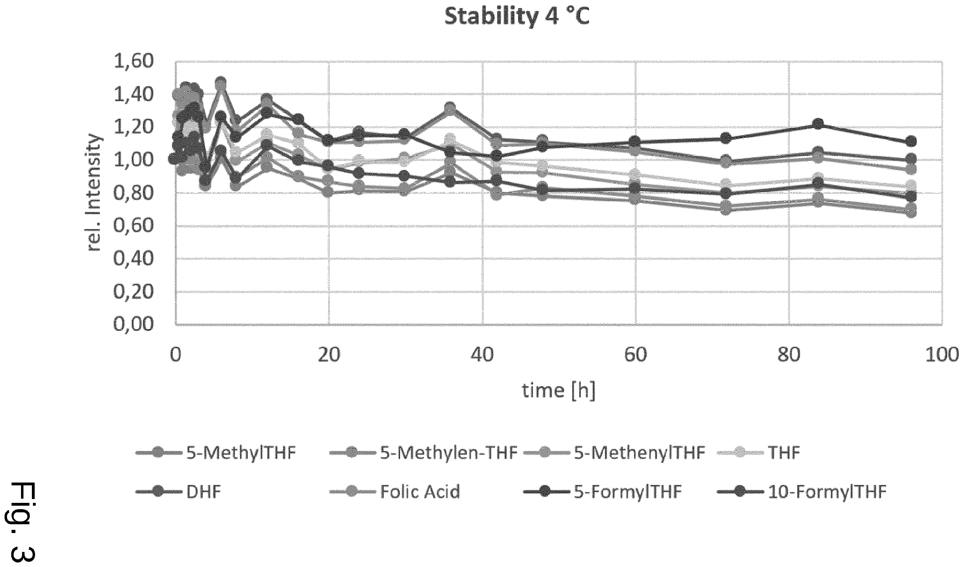

In contrast, the stabilised folate species showed excellent stability as can be seen in Fig. 3, under both storage (pH = 4.5) and chromatographic conditions (pH = 9.2).

In summary, stabilizing folates allowed to more reliably quantify individual folate species and permitted storage of samples before measurement.

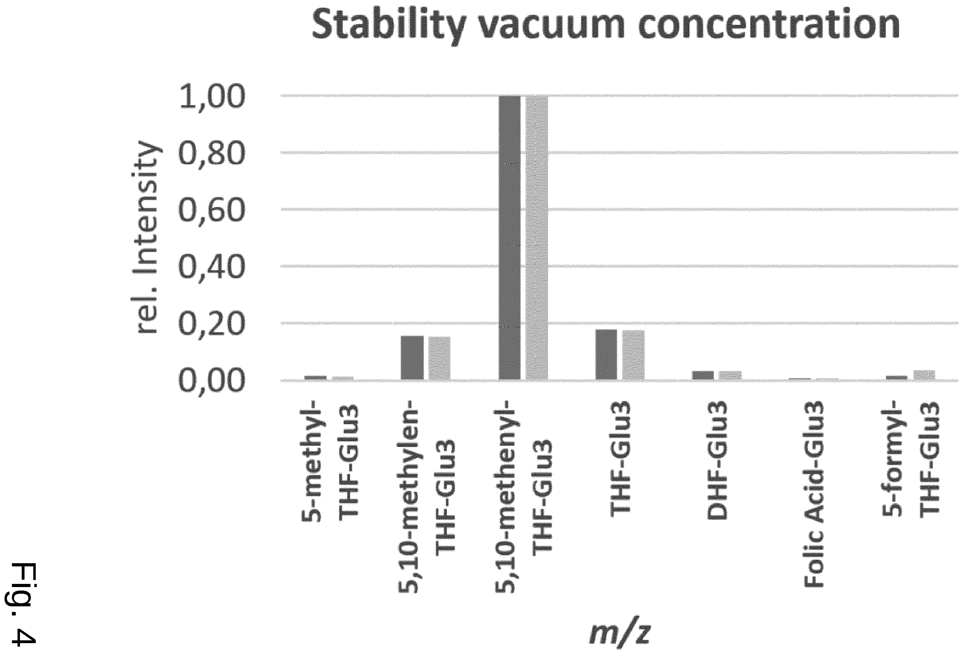

The present invention also enabled pre-concentration of biological extracts, e.g. by using a rotary vacuum concentrator while maintaining the ratios of individual folate species. Relative abundance of folate species in an *E. coli* extract stabilised according to the present invention remained unchanged pre- and post-concentration, as shown in Fig. 4.

### Example 5 - Testing sensitivity of LC-MS measurements of folate species stabilised according to the present invention

Folate species stabilised according to Example 1 were measured at different concentrations in LC-MS.

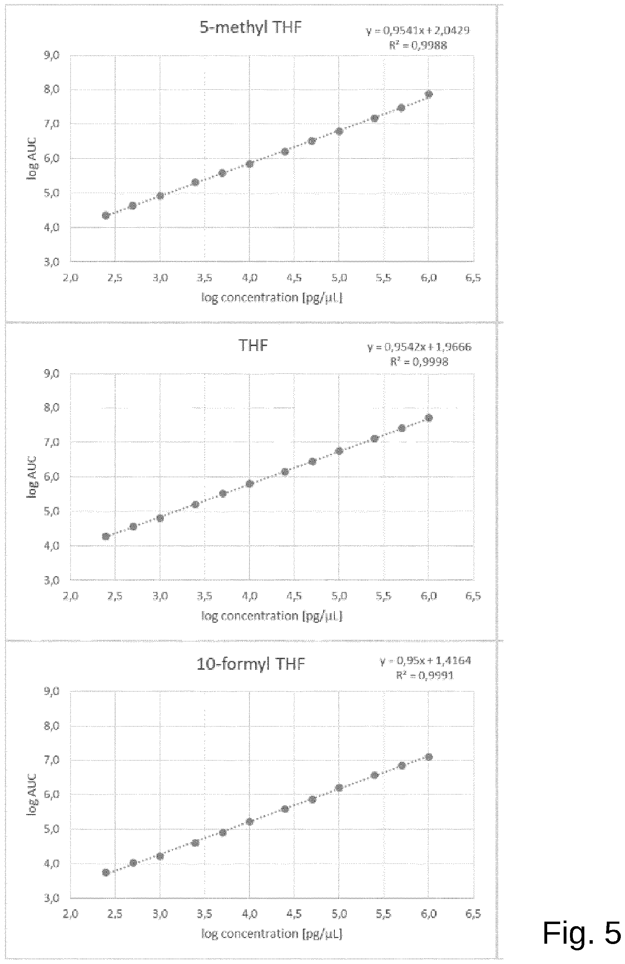

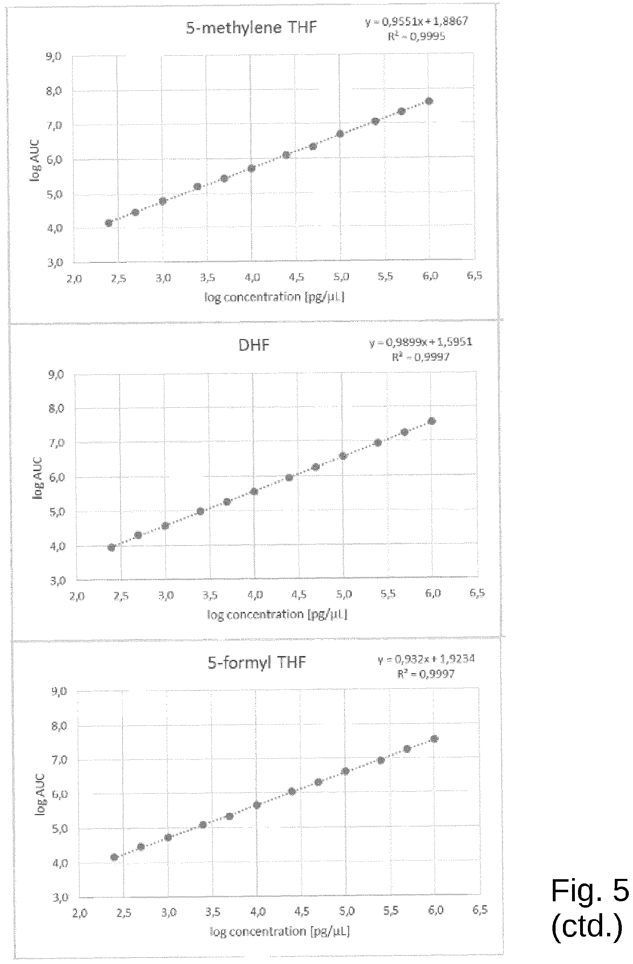

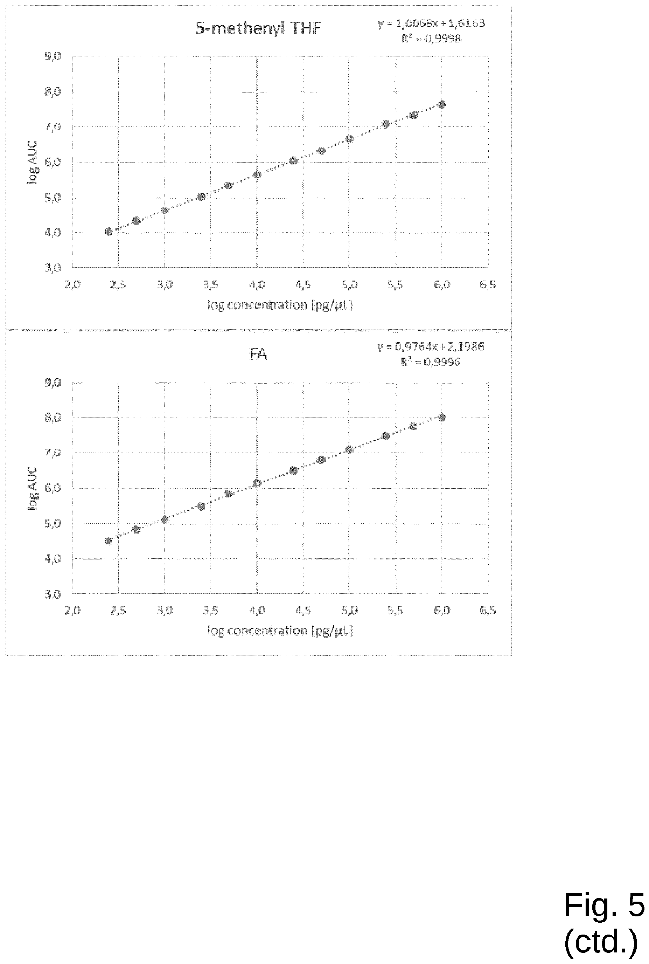

Compared to the methods of Lu et al. and Haandel et al., the sensitivity was improved for all monoglutamated folates, with the limit of detection (LOD, signal to noise > 5) being < 250 fg on column for all species. Linearity of quantification was excellent for almost 4 orders of magnitude with R² ≥ 0.99 for all analytes in the calibration range (250 fg to 1 µg) for both unweighted and double logarithmically weighted regression (see also Fig. 5).

### Example 6 - Testing matrix effects in connection with folate species stabilised according to the present invention

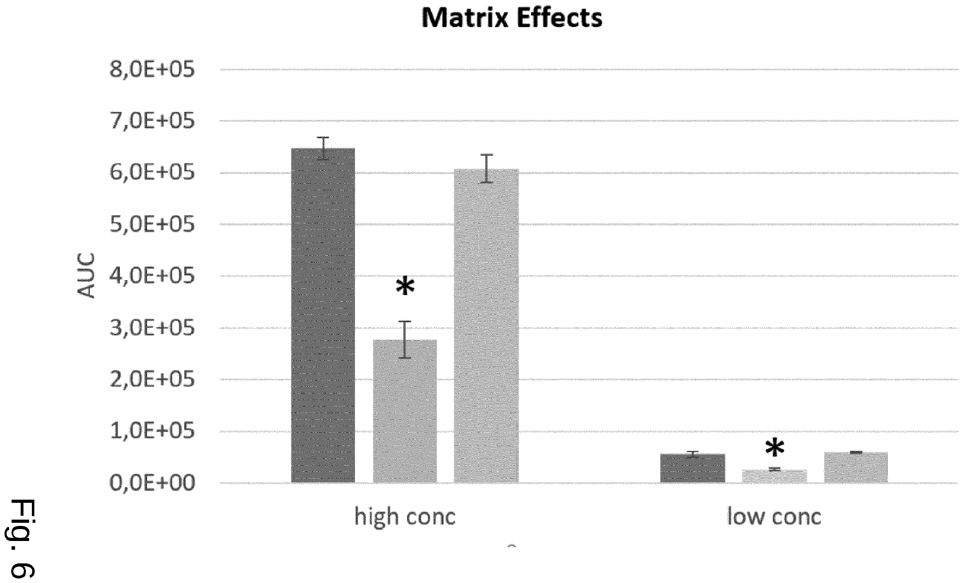

As matrix effects can play an important role in MS analysis, different ionization efficiencies in different backgrounds were tested by spiking two concentrations ("low conc", "high conc") of the light labelled dimethyl THF (Fig. 1B, compound B) into extraction buffer, *E. coli* extract and vacuum concentrated HepG2 cell extract, both stabilised according to the present invention. The area under curve is compared in Fig. 6. No significant matrix effect was observed in the vacuum concentrated HepG2 cell extract. A strong matrix effect was observed in E. coli lysate, signal intensities only amounted to 45 % of signal intensities in the spiked extraction buffer. Accordingly, for bacterial samples, adding an appropriate isotopic standard may be advantageous to account for the matrix effects.

In conclusion, therefore, the inventive method is particularly suitable for bacterial as well as mammalian, especially human, samples.

### Example 7 - Compatibility with metabolites up- and downstream of C1 pathway

Primary and secondary amines undergo reductive methylation under the employed stabilisation conditions disclosed in Example 1. Hence, amino acids such as glycine, serine and methionine as well as their derivatives e.g. S-adenosylmethionine (SAM) are di-methylated when stabilizing folate species. However, this does not prevent their quantification. No derivatization was observed for nucleotides and nucleotide building blocks. An overview of up- and downstream metabolites covered by the MRM method can be found in Fig. 1 and the complete set is also listed in Table 1.

### Example 8 - Applying the inventive stabilisation method to bacterial biological samples

50 mL LB-Miller medium was inoculated from an overnight culture of *E. coli* K12 to an OD600 of 0.1 and grown at 37 °C in a 500 mL flask shaking at 300 rpm. OD was measured at 0, 60, 90 min and immediately before harvesting. After reaching an OD of 0.4, trimethoprim was added to the treatment group (5 biological replicates) (0.15 mL of a 1 mg/mL stock solution in methanol; target concentration 10 µM) and the same volume of methanol was added to the control group (5 biological replicates). 30 min after adding trimethoprim, OD600 was measured (mean OD600 0.71 for control group and 0.63 for treated group) and cells were centrifuged at 4500 g for 3 min. The supernatant was removed.

Stabilisation by reductive alkylation according to the present invention: The cells were immediately resuspended in the stabilising liquid of 0.5 mL ice cold 80 % methanol containing 30 mM NaCNBD₃, 0.2 % Formaldehyde-¹³C, d2 and 0.1 % acetic acid by pipetting. Samples were incubated on ice for 60 min for the stabilisation of the folate species naturally present in the cells to continue.

Samples were then transferred to microcentrifuge tubes and centrifuged at 16,000 g for 1 min. Supernatants were transferred to a new tube and the pellet was resuspended in 0.5 mL ice cold 80 % methanol, 0.1 % acetic acid and incubated in sonic bath for 15 min. After sonication, the suspension was centrifuged at 16,000 g for 3 min and the supernatant was combined with the previous supernatant. An aliquot of 50 µL was subjected to MS analysis according to Example 2 (not concentrated sample). The remaining 950 µL were dried in a rotary evaporator and resuspended by sonication in 50 µL 80 % methanol, 0.1 % acetic acid for 15 min. Samples were centrifuged at 16,000 g for 3 min and the supernatant was analysed in MS according to Example 2 (concentrated sample).

### LC-MS results:

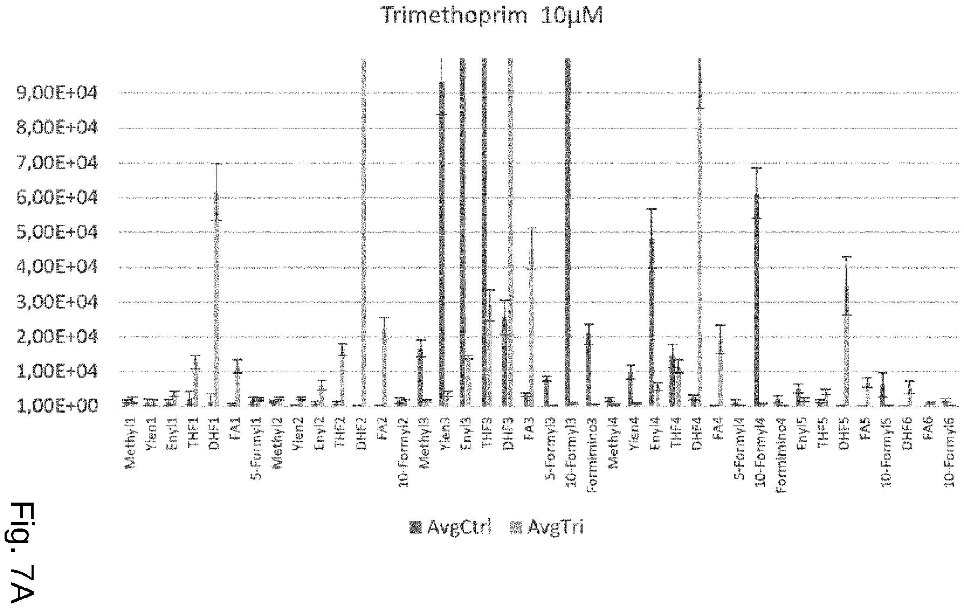

The distribution of folypolyglutamates with n = 3 being most abundant in control cells, followed by n = 4 and n = 5 (see Fig. 7A), was similar to the results of Lu et al. who performed a similar trimethoprim inhibition experiment (yet without the inventive stabilisation). Trimethoprim is an inhibitor of dihydrofolate reductase (DHFR), which reduces both FA (via DHF) and DHF to THF. DHFR activity is not only necessary for de novo synthesis of folates but also to recycle DHF which is produced from 5,10-methylene THF during thymidine synthesis. As expected, treatment with trimethoprim resulted in a marked increase of folates with oxidized pteridine ring (DHF, FA), while all poly-glutamated species with a reduced ring were depleted. This served as a validation that the inventive stabilisation method still produced biologically meaningful results.

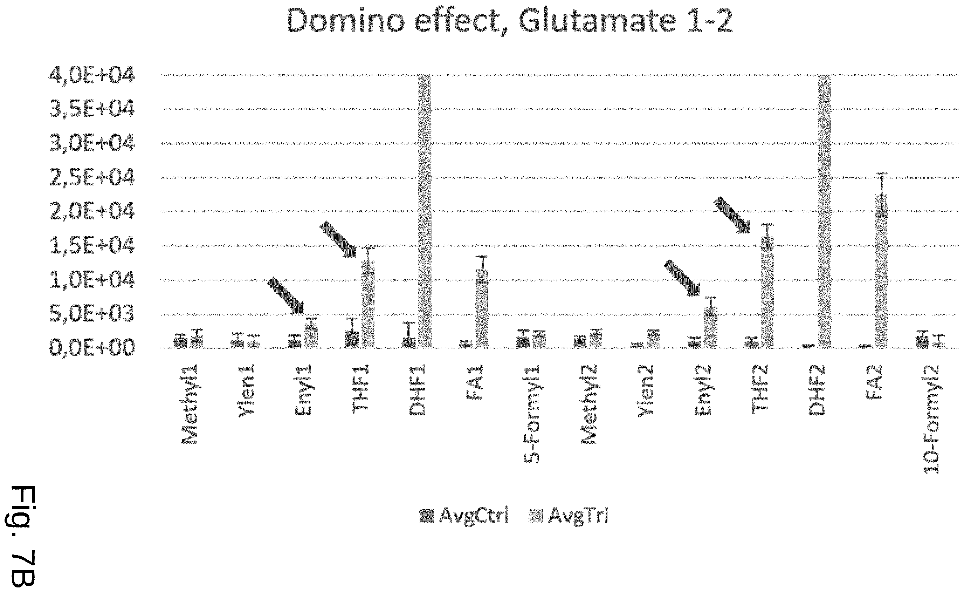

Despite stabilisation, also the "domino effect" described by Kwon et al. could be observed. The "domino effect" relates to the increase of reduced folates with low glutamation number (n = 1-2) after prolonged treatment (in this case 30 min) with trimethoprim. THF and 5,10-methenyl THF showed the most pronounced domino effect (Fig. 7B, red arrows).

The folate profile of the control group grown on complex medium differed markedly from the profile reported for minimal medium (the latter of which was performed by Lu et al.). While the fully reduced 5-methyl THF was reported to be the dominant folate species on minimal medium, 5,10-methenyl THF was the most abundant folate species in *E. coli* grown in complex medium, followed by 10-formyl THF. With the capability of the inventive method to distinguish between 5- and 10-formyl THF it could also be concluded that poly-glutamated 5-formyl THF was a minor species under the employed conditions, with only poly-glutamated FA being lower.

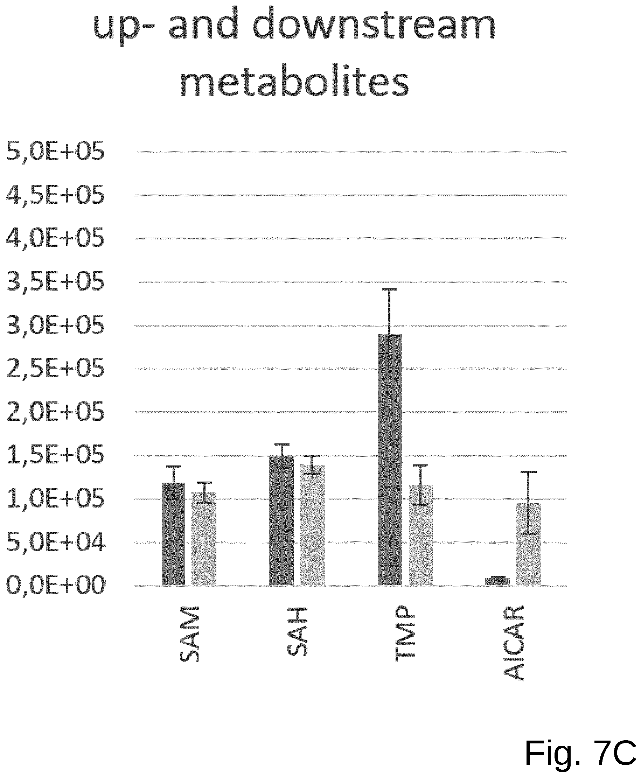

Substrates and products of C1 metabolism were also monitored. It was found that thymidine was significantly downregulated in treated cells while the purine base biosynthetic intermediate AICAR was significantly enriched (Fig. 7C, p < 0.001 for both), both in agreement with a lack of biosynthetically available C1 units. Interestingly, the SAM to S-adenosyhomocysteine (SAH) ratio was not altered, indicating a precedence of the methylation system over de *novo* nucleotide synthesis.

### Example 9 - Applying the inventive stabilisation method to a biological sample from human cells

Inhibitors of the folate cycle are widely employed in chemotherapeutic treatment of cancer (Gonen et al.). Human liver cancer cell line HepG2 was therefore treated with methotrexate, which inhibits DHFR as trimethoprim does in bacteria:
HepG2 cells were acquired from ATCC (ATCC® HB-8065™) and grown in T75 flasks in RPMI1640 (Gibco, Thermo Fisher Scientific, Switzerland) containing 2 mM Glutamine, 2 g/L glucose and 10 % FBS. 1 mg Methotrexate hydrate (Sigma Aldrich, Buchs SG, Switzerland) was dissolved in 10 µL 1 M sodium hydroxide and diluted with phosphate buffered saline to 1 mL and sterile filtered (2.2 mM Stock solution). At 80 % cell confluence, 20 µM Methotrexate was added to the treatment group (180 µL stock solution per 20 mL medium). 180 µL of phosphate buffered saline containing 1 % (v/v) 1 M sodium hydroxide was added to the control group. 16 h post treatment, cells were harvested by trypsinization and centrifuged at 4 °C and 400 g for 5 min. The supernatant was removed and the cell pellet was briefly washed with 1 mL PBS.

Stabilisation by reductive alkylation according to the present invention: The cell pellet was then resuspended in 0.5 mL ice cold 80 % methanol, containing 30 mM NaCNBD₃, 0.2 % Formaldehyde-13C, d2 and 0.1 % acetic acid by pipetting. Samples were incubated on ice for 60 min for the stabilisation of the folate species naturally present in the cells to continue.

Subsequent steps were carried out as described for *E.coli* in Example 8.

### LC-MS results:

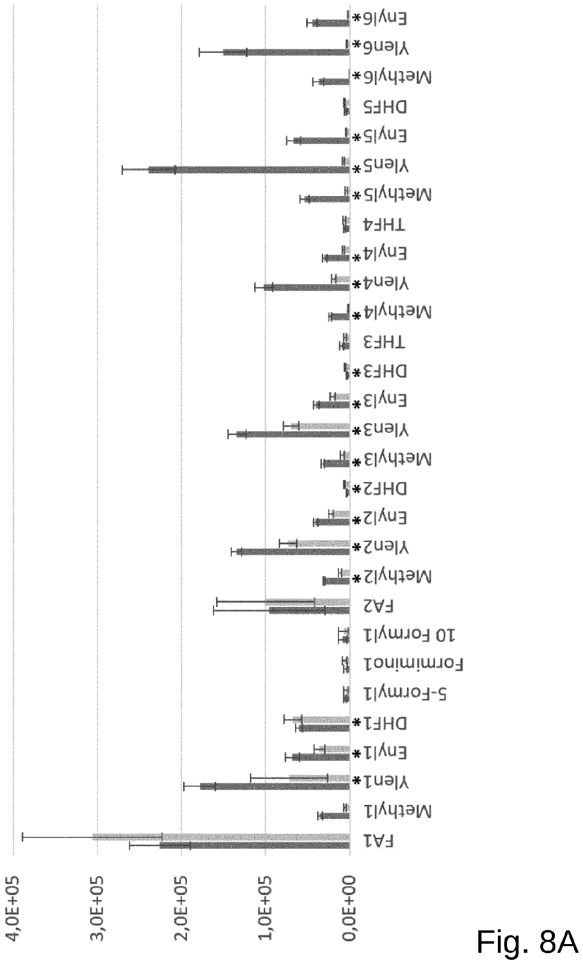

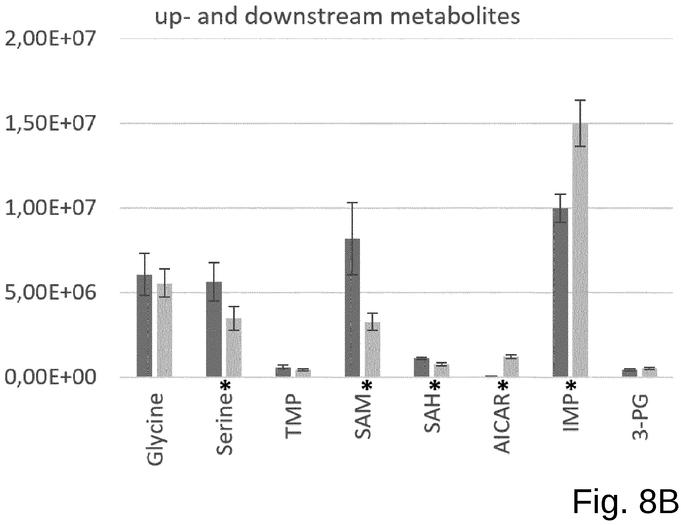

In contrast to the bacterial system, no significant increase of the oxidized pteridines was observed with the exception of DHF-Glu2. However, most of the reduced folates were significantly reduced (see Fig. 8A). No domino effect was observed after 16 h of treatment with methotrexate. Interestingly, serine was significantly reduced in the treated group (3 biological replicates) compared to control (see Fig. 8B). While the levels of thymidine did not change, both AICAR and inosine monophosphate were upregulated upon methotrexate treatment. Finally, both SAM and SAH as well as their ratio were reduced significantly.

In conclusion, the present invention is suitable for folate analysis of both bacterial as well as mammalian biological samples.

### Non-patent references

Assaraf, Y. G., Cancer and Metastasis Reviews 2007, 26 (1), 153-181.
Boersema, Paul J., et al. "Multiplex peptide stable isotope dimethyl labeling for quantitative proteomics." Nature protocols 4.4 (2009): 484.
Chen, Li, et al. Analytical and bioanalytical chemistry 409.25 (2017): 5955-5964.
Fang, Houqin, et al. "Intact Protein Quantitation Using Pseudoisobaric Dimethyl Labeling." Analytical chemistry 88.14 (2016): 7198-7205.
Freisleben, A.; Schieberle, P.; Rychlik, M., Analytical and Bioanalytical Chemistry 2003, 376 (2), 149-156.
Garratt, L. C.; Ortori, C. A.; Tucker, G. A.; Sablitzky, F.; Bennett, M. J.; Barrett, D. A., Rapid Communications in Mass Spectrometry 2005, 19 (17), 2390-2398.
Gonen, N.; Assaraf, Y. G., Drug Resistance Updates 2012, 15 (4), 183-210.
Haandel, L.; Becker, M. L.; Williams, T.; Stobaugh, J.; Leeder, J. S., Rapid Communications in Mass Spectrometry 2012, 26, 1617-1630.
Iacobazzi, V.; Castegna, A.; Infantino, V.; Andria, G., Molecular Genetics and Metabolism 2013, 110 (1-2), 25-34.
Jägerstad, M.; Jastrebova, J., Journal of Agricultural and Food Chemistry 2013, 61 (41), 9758-9768.
Kiekens, F.; Daele, J. V.; Blancquaert, D.; Van Der Straeten, D.; Lambert, W. E.; Stove, C. P., Journal of Agricultural and Food Chemistry 2015, 63 (45), 10089-10095.
Kovanich, Duangnapa, et al. "Applications of stable isotope dimethyl labeling in quantitative proteomics." Analytical and bioanalytical chemistry 404.4 (2012): 991-1009.
Kwon, Y. K.; Lu, W.; Melamud, E.; Khanam, N.; Bognar, A.; Rabinowitz, J. D., Nature Chemical Biology 2008, 4 (10), 602-608.
Li, Y.; Huang, T.; Zheng, Y.; Muka, T.; Troup, J.; Hu, F. B., Journal of the American Heart Association 2016, 5 (8).
Locasale, J. W., Nature Reviews Cancer 2013, 13 (8), 572-583.
Lu, W.; Kwon, Y. K.; Rabinowitz, J. D., Journal of the American Society for Mass Spectrometry 2007, 18 (5), 898-909.
Narisawa, A.; Komatsuzaki, S.; Kikuchi, A.; Niihori, T.; Aoki, Y.; Fujiwara, K.; Tanemura, M.; Hata, A.; Suzuki, Y.; Relton, C. L.; Grinham, J.; Leung, K.-Y.; Partridge, D.; Robinson, A.; Stone, V.; Gustavsson, P.; Stanier, P.; Copp, A. J.; Greene, N. D. E.; Tominaga, T.; Matsubara, Y.; Kure, S., Human Molecular Genetics 2012, 21 (7), 1496-1503.
Newman, A. C.; Maddocks, O. D. K., British Journal of Cancer 2017, 116 (12), 1499-1504.
Reed, L. S.; Archer, M. C., Journal of Agricultural and Food Chemistry 1980, 28 (4), 801-805.
Ringling, C.; Rychlik, M., European Food Research and Technology 2013, 236 (1), 17-28.
Ringling, C.; Rychlik, M., Analytical and Bioanalytical Chemistry 2017, 409 (7), 1815-1825.
Schittmayer, M.; Fritz, K.; Liesinger, L.; Griss, J.; Birner-Gruenberger, R., Journal of Proteome Research 2016, 15 (4), 1222-9
Strandler, H. S.; Patring, J.; Jägerstad, M.; Jastrebova, J., Journal of Agricultural and Food Chemistry 2015, 63 (9), 2367-2377.
Stover, P.; Schirch, V., Analytical Biochemistry 1992, 202 (1), 82-88.

## Claims

1. A method of stabilising a biological sample comprising at least two folate species, the method comprising the following steps:
- providing the biological sample comprising at least two folate species, and
- contacting the at least two folate species with a reducing agent and an aliphatic aldehyde or ketone under conditions which allow reductive alkylation of at least one of the at least two folate species, wherein the reducing agent is a deuterated reducing agent and the aliphatic aldehyde or ketone is a deuterated aliphatic aldehyde or ketone, whereby at least one stabilised folate species isotopically labelled with D is obtained.

2. The method of claim 1, wherein the aliphatic aldehyde or ketone has a ¹³C carbon atom, wherein the at least one stabilised folate species is obtained further isotopically labelled with ¹³C.

3. The method of claim 1 or 2, wherein the reducing agent is a cyanoborodeuteride.

4. The method of any one of claims 1 to 3, wherein the aliphatic aldehyde or ketone is deuterated formaldehyde, acetaldehyde, propionaldehyde or butyraldehyde.

5. The method of claim 4, wherein the aliphatic aldehyde or ketone is formaldehyde-¹³C, D2.

6. The method of any one of claims 1 to 5, wherein the biological sample comprises cells which comprise the at least two folate species, preferably wherein the biological sample is a tissue sample, in particular a biopsy sample.

7. The method of any one of claims 1 to 6, further comprising the step of adding a liquid comprising an organic solvent to the biological sample before said contacting step, preferably wherein the liquid contains the reducing agent and the aliphatic aldehyde or ketone.

8. The method of claim 7, wherein the liquid further comprises an acid, preferably an organic acid such as acetic acid, formic acid or propionic acid or a mixture thereof, and has a pH between 0 and 6.75, preferably between 1 and 6, more preferably between 2 and 5, even more preferably between 3 and 5, yet even more preferably between 4 and 5, in particular between 4.25 and 4.75.

9. The method of claim 7 or 8, wherein the liquid is added directly to the cells, preferably the tissue sample, in particular the biopsy sample.

10. The method of any one of claims 1 to 9, further comprising the step of concentrating and/or drying the sample, or a fraction thereof, containing the at least one stabilised folate species.

11. A biological sample containing at least one stabilised folate species isotopically labelled with D and preferably with ¹³C, obtainable by the method of any one of claims 1 to 10.

12. A biological sample or standard for mass spectrometry containing at least one stabilised folate species selected from the group consisting of compounds I-VIII: 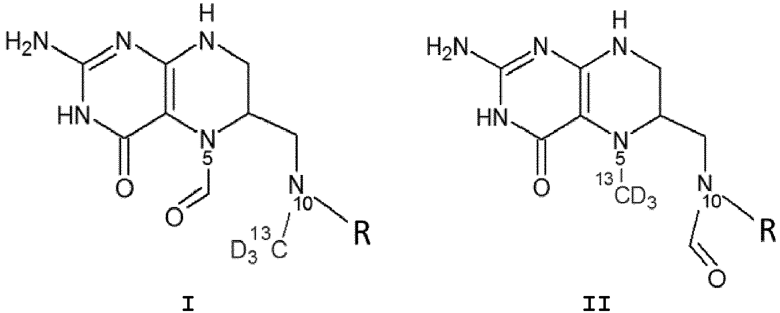 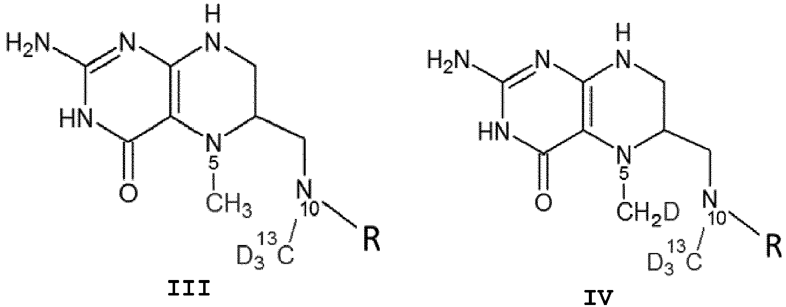 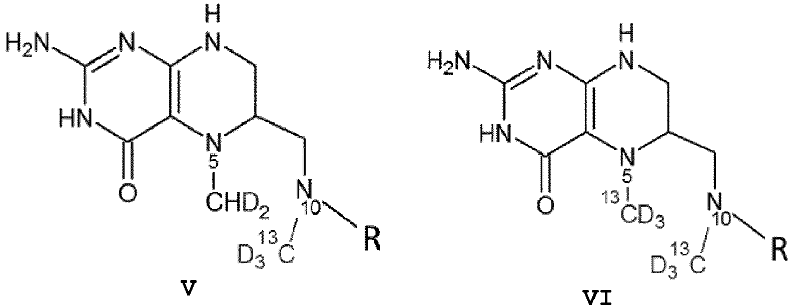 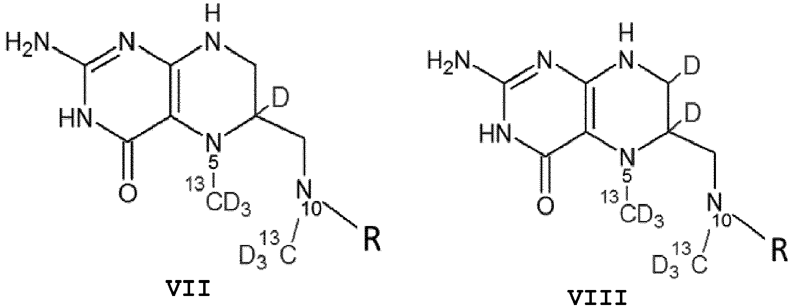 wherein R is 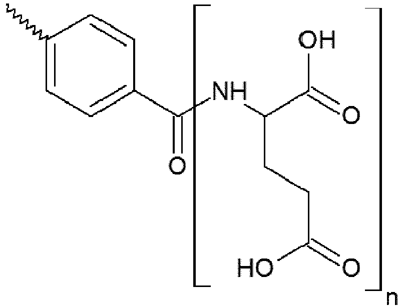 wherein n is an integral from 1 to 8.

13. A method for quantifying folate species in a biological sample, the method comprising the following steps:
- providing a biological sample as defined in claim 11 or 12,
- optionally, purifying the biological sample in a liquid chromatography system, and
- analysing at least a fraction of the biological sample, which fraction contains at least a portion of the at least one stabilised folate species, in a mass spectrometer to measure the abundance of the at least one stabilised folate species.

14. A kit for stabilising a biological sample comprising at least two folate species for mass spectrometric analysis, the kit comprising
- a first container containing a reducing agent, wherein the reducing agent is deuterated,
- a second container containing an aliphatic aldehyde or ketone, wherein the aliphatic aldehyde or ketone is deuterated, and
- a third container containing a liquid comprising an organic solvent;
wherein the liquid further contains an acid and/or an acid is provided in a fourth container.

15. The kit of claim 14, wherein the reducing agent is a cyanoborodeuteride.
